# EUROPÄISCHE PATENTSCHRIFT

(11) **EP 0 466 164 B1**
(45) Veröffentlichungstag und Bekanntmachung des Hinweises auf die Patenterteilung: **07.06.1995**
(21) Anmeldenummer: 91111597.0
(22) Anmeldetag: 12.07.1991
(51) Int. Cl.: C07D 473/40

(54) **Verfahren zur Herstellung 2-Chlor-1,7-dihydropurin-6-on und Verfahren zu dessen Reinigung**
Process for the preparation of 2-chloro-1,7-dihydropurin-6-one and process for the purification of the same
Procédé pour la préparation de 2-chloro-1,7-dihydropurin-6-one et procédé pour sa purification

(30) Priorität: 13.07.1990 DE 4022314
(43) Veröffentlichungstag der Anmeldung: 15.01.1992
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Ramert, Reiner, Dr. Dipl.-Chem., W-6531 Weiler bei Bingen (DE); Christmann, Albrecht, Dr. Dipl.-Chem., W-6507 Ingelheim am Rhein (DE)

(56) Entgegenhaltungen:
- DE-C- 96 925
- CHEMICAL ABSTRACTS, vol. 66, no. 21, 22. Mai 1967, Columbus, Ohio, US; abstractno. 95367Z, YOSHITAKA YAMADA E. A.: '2-Chlorohypoxanthines.' Seite 8953 ;Spalte2 ;
- JOURNAL OF THE AMERICAN CHEMICAL SOCIETY Bd. 79, Nr. 9, 7. Mai 1957, WASHINGTOND. C. Seiten 2185 - 2188; JOHN A. MONTGOMERY E. A.: 'Synthesis of PotentialAntcancer Agents.'
- LIEBIGS ANNALEN DER CHEMIE Bd. 649, 1961, WEINHEIM Seiten 114 - 125; HUBERTBALLWEG: 'Purinderivate des Histamins'

## Beschreibung

Die Erfindung betrifft ein Verfahren zur Herstellung und Reinigung von 2-Chlor-1,7-dihydropurin-6-on (2-Chlorhypoxanthin). 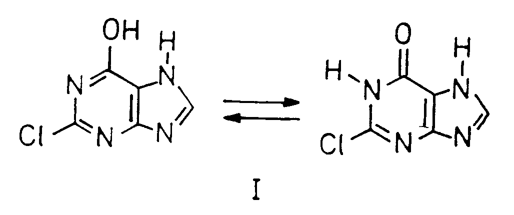
2-Chlorhypoxanthin (I) leitet sich vom Grundkörper des 9H-Purins ab (welches bei unsubstituierter 7- und 9-Stellung mit seinem Tautomeren, dem 7H-Purin, im Gleichgewicht steht [H. Beyer und W. Walter, Lehrbuch der organischen Chemie, S. Hirzel Verlag, Stuttgart 1988, S. 797]).

Derivate der Purine haben in den vergangenen Jahren eine stürmische Entwicklung hinsichtlich ihres Einsatzes als Therapeutika erfahren [T.W. Stone Ed., Purines - Pharmacology and Physiological Roles, VCH Verlagsgesellschaft, Weinheim 1985].

So besitzt eine große Zahl von Nucleosiden, die eine Purin-Teilstruktur aufweisen, antimetabolische Eigenschaften. Dabei konnten einige Nucleosid-Derivate gefunden werden, welche in der Lage sind, die Vermehrung des für die AIDS-Erkrankung verantwortlichen HIV-Virus einzuschränken.

Zu dieser Gruppe wirksamer Nucleoside gehören insbesondere Nucleoside, in welchen die sog. Nucleosid-Base einen natürlich vorkommenden oder derivatisierten Purinkörper aufweist. Besonders aussichtsreich erscheint dabei der Einsatz von Purinabkömmlingen die am Stickstoffatom in 9-Position derivatisiert und in der 2-Position funktionalisiert sind [s.a. Europäische Patentanmeldung 343 133 und 291 917]. Als Ausgangsstoff für derartig derivatisierte Purine bietet sich das 2-Chlorhypoxanthin (I) an, welches schon über eine geeignete Funktionalisierung der 2-Position verfügt.

Die bisher zur Herstellung dieser attraktiven Zwischenverbindung literaturbekannten Verfahren leiden jedoch entweder unter dem Mangel, daß die industrielle Herstellung nur unter großem Aufwand durchführbar ist, oder daß die notwendigen Edukte nicht kommerziell verfügbar sind.

So beschreiben J.A. Montgomery und L.B. Holum [J. Am. Chem. Soc. 79 (1957) 2185] einen Syntheseweg ausgehend von 2,6-Dichlorpurin. Ein ähnliches Verfahren beschreiben Y. Yamada et al. [Chemical Abstracts 66 (1967) 9536 8a]. Die Nachteile beider Verfahrensweisen bestehen insbesondere darin, daß die partielle Hydrolyse der Dichlorverbindung eine hohe Verdünnung des Ausgangsmaterials erforderlich macht und das 2-Chlorhypoxanthin lediglich in einer Ausbeute von 66 % (Rohprodukt) isoliert werden kann. Außerdem ist 2,6-Dichlorpurin teuer, kommerziell nicht in großen Mengen verfügbar und wegen seiner akuten Reizwirkung sowie seiner allergenen, potentiell kanzerogenen Eigenschaften ein Arbeitsstoff, dessen Handhabung erhebliche sicherheitstechnische und arbeitshygienische Probleme aufwirft. Diese nachteiligen Aspekte lassen eine derartige Syntheseroute für eine industrielle Herstellung von 2-Chlorhypoxanthin als unbrauchbar erscheinen.

Das zweite in der Literatur beschriebene Verfahren geht von 2,8-Dichlor-6-hydroxypurin aus [E. Fischer, Chem. Ber. 30 (1897) 2208; ibid. 30 (1897) 2226], welches jedoch nicht kommerziell verfügbar ist und seinerseits erst aus Harnsäure und Phosphoroxychlorid hergestellt werden muß.

Das so synthetisierte 2,8-Dichlor-6-hydroxypurin muß in einem weiteren Reaktionsschritt entweder mit Jodwasserstoff oder auf dem Wege der selektiven katalytischen Hydrogenolyse [H. Ballweg, Liebigs Ann. Chem. 649 (1961) 114] partiell dehalogeniert werden. Während die Dehalogenierung mit Jodwasserstoff sehr aufwendig und für eine industrielle Anwendung unbrauchbar ist, muß bei der katalytischen Hydrogenolyse das Reaktionsprodukt mehrmals aus Wasser umkristallisiert werden und fällt danach in einer Ausbeute von lediglich 77 % an, womit auch dieses Verfahren für eine Übertragung in einen industriellen Maßstab ungeeignet ist.

Daneben müssen an die Reinheitskriterien von Zwischenprodukten, die in Herstellungsverfahren zur Synthese von Pharmazeutika eingesetzt werden, naturgemäß sehr hohe Anforderungen gestellt werden.

Die Aufgabe der vorliegenden Erfindung besteht darin, ein Verfahren zur Herstellung von 2-Chlorhypoxanthin (I) (2-Chlor-1,7-dihydropurin-6-on) zur Verfügung zu stellen, welches die Herstellung dieses Purinderivates in einfacher Weise und in guten Ausbeuten erlaubt.

Eine weitere Aufgabe der vorliegenden Erfindung besteht darin, ein Syntheseverfahren zur Verfügung zu stellen, bei dem die Reaktions- bzw. Zwischenprodukte in einer kristallinen Form anfallen, die eine unproblematische Weiterverarbeitung - Schleudern, Trocknen usw. - auch bei großtechnischen Ansatzgrößen ermöglicht.

Eine weitere Aufgabe der vorliegenden Erfindung besteht darin, ein Reinigungsverfahren zur Verfügung zu stellen, bei welchem das 2-Chlorhypoxanthin (I) in einer Reinheit anfällt, die es erlaubt, das Produkt direkt als Ausgangsstoff in weitere Verfahren zur Herstellung von pharmazeutisch wirksamen Substanzen einzusetzen.

### Herstellung des 2-Chlorhypoxanthin-Hydrochlorid-Monohydrates

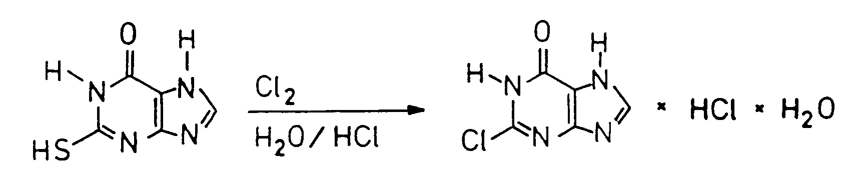

Erfindungsgemäß werden die vorgenannten Aufgaben dadurch gelöst, daß man ausgehend von käuflichem 2-Thioxanthin durch Chlorierung in Gegenwart von konzentrierter Salzsäure zunächst das Monohydrat des 2-Chlorhypoxanthin-Hydrochlorids herstellt. Dazu wird das 2-Thioxanthin zunächst in konzentrierter Salzsäure suspendiert und in die Reaktionsmischung über einen Zeitraum von 3 bis 7 Stunden - bevorzugt ca. 5 Stunden - bei einer Temperatur von 0°C bis 10°C - bevorzugt 2°C bis 7°C und besonders bevorzugt 3°C bis 5°C - Chlor eingeleitet. Anschließend wird ein Teil der Salzsäure abdestilliert, der Rückstand auf eine Temperatur im Bereich von -5°C bis +10°C - bevorzugt 0°C bis 5°C - abgekühlt, abgesaugt und der kristalline Rückstand zunächst mit Kochsalzlösung und anschließend mit einem organischen Lösungsmittel - bevorzugt einem Alkohol, besonders bevorzugt mit Isopropanol - gewaschen und getrocknet.

### Herstellung des 2-Chlorhypoxanthins

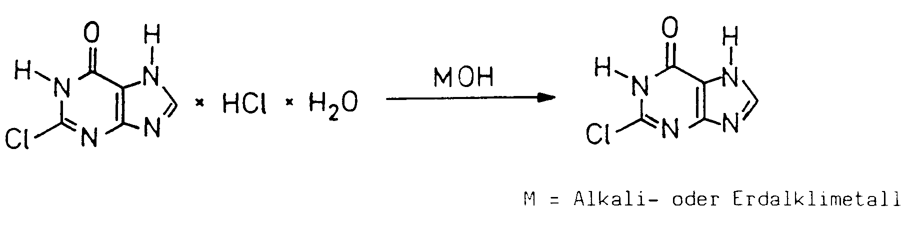

In der sich daran anschließenden Reaktionsstufe wird in der Suspension aus 2-Chlorhypoxanthin-Hydrochlorid-Monohydrat mit einer wäßrigen Lösung eines Alkali- oder Erdalkalihydroxids - vorzugsweise eines Alkalihydroxids und besonders bevorzugt mit konzentrierter Natronlauge ein pH-Wert im Bereich von 6 bis 7 - bevorzugt einen pH-Wert von 6,5 - eingestellt. Nach dem Erwärmen der Suspension auf eine Temperatur im Bereich von 50°C bis 70°C - bevorzugt 55°C bis 65°C und besonders bevorzugt auf eine Temperatur von 60°C - wird die entstandene Lösung - falls erforderlich - mit Entfärbungskohle behandelt und filtriert. In der wäßrigen Lösung des Filtrates wird mit der wäßrigen Lösung einer Säure - vorzugsweise einer Mineralsäure und besonders bevorzugt mit 1 N Salzsäure - ein pH-Wert im Bereich von pH 2 bis pH 4 - bevorzugt pH 3,0 - eingestellt.

Die resultierende Suspension wird auf eine Temperatur im Bereich von -5°C bis +15°C - bevorzugt 5°C bis 10°C - abgekühlt und der Niederschlag mit Wasser und anschließend mit einem organischen Lösungsmittel - bevorzugt einem Alkohol, besonders bevorzugt Isopropanol - gewaschen und getrocknet.

### Herstellung von Alkalisalzen des 2-Chlorhypoxanthins

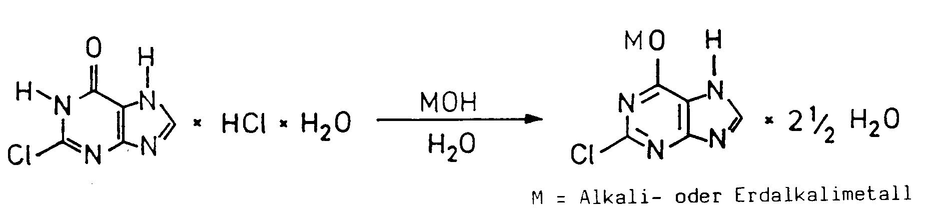

Zur Herstellung von hochreinem 2-Chlorhypoxanthin wird 2-Chlorhypoxanthin-Hydrochlorid-Monohydrat in Wasser suspendiert. Unter Kühlung wird mit der wäßrigen Lösung einer anorganischen Base - vorzugsweise einer Lösung eines Alkalihydroxids und besonders bevorzugt mit konzentrierter Natronlauge - in der Suspension ein pH-Wert im Bereich von 9 bis 10, bevorzugt 9,5 bis 10 und besonders bevorzugt 9,7 eingestellt.

Nach dem Abkühlen der Reaktionsmischung auf eine Temperatur unterhalb Raumtemperatur - bevorzugt auf eine Temperatur in einem Intervall von -2°C bis +10°C, besonders bevorzugt 2°C bis 5°C - wird der dabei ausfallende kristalline Niederschlag abgesaugt und zunächst mit gekühlter Kochsalzlösung und darauf mit einem organischen Lösungsmittel - bevorzugt einem Alkohol und besonders bevorzugt Isopropanol - gewaschen und getrocknet.

### Herstellung von hochreinem 2-Chlorhypoxanthin (I)

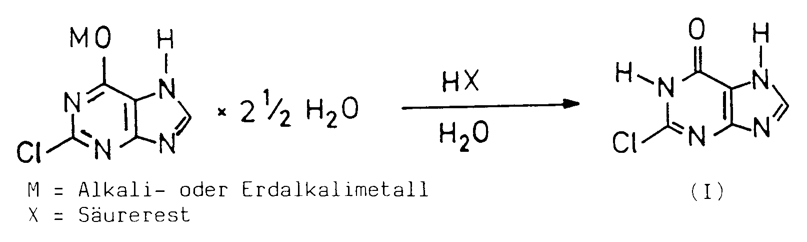

In einer Suspension von 2-Chlorhypoxanthin-Natrium-Hydrat in Wasser wird mit Säure - vorzugsweise mit einer Mineralsäure und besonders bevorzugt mit verdünnter Salzsäure ein pH-Wert im Bereich von pH 6 bis pH 7 - bevorzugt pH 6,5 - eingestellt. Anschließend wird die Suspension auf eine Temperatur im Bereich von 55°C bis 65°C - vorzugsweise 60°C erwärmt.

Die resultierende Lösung wird - gegebenenfalls nach der Behandlung mit Entfärbungskohle - filtriert. In der wäßrigen Lösung des Filtrates wird mit der wäßrigen Lösung einer Säure - vorzugsweise einer Mineralsäure und besonders bevorzugt mit 1 N Salzsäure - ein pH-Wert im Bereich von pH 2 bis pH 4 - bevorzugt pH 3,0 - aufrechterhalten. Die resultierende Suspension wird anschließend auf eine Temperatur unterhalb Raumtemperatur - vorzugsweise auf eine Temperatur im Intervall von -2°C bis +15°C, besonders bevorzugt 5 bis 10°C - abgekühlt. Der dabei ausfallende Niederschlag wird abgesaugt und mit Wasser und anschließend mit einem organischen Lösungsmittel - vorzugsweise einem Alkohol und besonders bevorzugt Isopropanol - gewaschen.

Der kristalline Rückstand wird getrocknet. Das auf diese Art und Weise gewonnene 2-Chlorhypoxanthin weist - laut Aussage der HPLC-Analyse - eine Reinheit von über 99% auf.

Die vorstehend erläuterten Verfahrensschritte werden durch die in den Beispielen genannte Reaktionsfolge genauer beschrieben. Verschiedenartige, andere Ausgestaltungen des Verfahrens und dergleichen werden für den Fachmann aus der vorliegenden Beschreibung ersichtlich.

### Beispiele

### 1) Herstellung von 2-Chlorhypoxanthin-Hydrochlorid-Monohydrat

In eine Suspension von 168,2 g 1,0 mol 2-Thioxanthin in 1,68 l konz. Salzsäure werden im Verlauf von ca. 5 h bei 3-5°C 230 g (3,24 mol) Chlor eingeleitet. Danach wird im Wasserstrahlvakuum ca. 1 l Salzsäure abdestilliert, der Rückstand auf 0 bis + 5°C abgekühlt, abgesaugt und mit 0,5 l gesättigter, eiskalter Kochsalzlösung sowie abschließend mit 300 ml Isopropanol gewaschen. Nach Trocknen bei 50°C über einen Zeitraum von ca. 12 h resultieren 193 g (85,7 % d. Th.) farbloses Kristallpulver. Durch Herstellen der freien Base und erneutes Umwandeln in das Hydrochlorid wird die Titelverbindung in analysenreiner Form erhalten. Die Elementaranalyse entspricht der Zusammensetzung C₅H₄Cl₂N₄O x H₂O.

### 2) Herstellung von 2-Chlorhypoxanthin

180 g (0,80 mol) 2-Chlorhypoxanthin-Hydrochlorid-Monohydrat werden in 3,6 l Wasser suspendiert und der pH-Wert durch Zutropfen von konzentrierter Natronlauge auf pH = 6,5 eingestellt. Nach Erwärmen auf 60°C wird die Lösung mit 18 g Entfärbungskohle behandelt und filtriert. Das Filtrat wird in 300 ml Wasser getropft, wobei in der wäßrigen Lösung mit 1N Salzsäure ein pH-Wert von 3,0 +/- 0,5 aufrechterhalten wird. Die resultierende Suspension wird auf 5-10°C abgekühlt, abgesaugt und der Niederschlag mit 0,5 l Wasser mit einer Temperatur von + 5°C sowie 300 ml Isopropanol gewaschen. Nach dem Trocknen bei 50°C werden 117 g (85,8 % d. Th.) der Titelverbindung in Form eines farblose Kristallpulvers erhalten. Durch Reinigung über das Natriumsalz und erneutes Freisetzen der Base wird analysenreines Material erhalten, das mit der nach J.A. Montgomery und L.B. Holum, [J. Am. Chem. Soc. 79 (1957) 2185] aus 2,6-Dichlorpurin hergestellten Substanz identisch ist.

### 3) Herstellung von 2-Chlorhypoxanthin-Natrium

80 g (0,31 mol) 2-Chlorhypoxanthin-Hydrochlorid-Monohydrat werden in 1,6 l Wasser suspendiert. Unter Kühlung wird mit konzentrierter Natronlauge ein pH-Wert von pH 9,7 eingestellt. Nach Abkühlen auf + 5°C wird der Niederschlag abgesaugt, mit 250 ml 20 %-iger Kochsalzlösung - vorgekühlt auf + 5°C - und 250 ml Isopropanol gewaschen. Nach Trocknen bei 50°C werden 65,7 g (77,6 % d. Th.) der Titelverbindung als farbloses Kristallpulver erhalten. Durch Suspendieren in kaltem Wasser und erneutes Absaugen, Waschen mit kaltem Wasser und Methanol sowie Trocknen wird ein analysenreines Produkt erhalten, dessen Zusammensetzung nach Aussage der Elementaranalyse der Formel C₅H₂ClN₄0 Na x 2,5 H₂0 entspricht.

### 4) Herstellung von hochreinem 2-Chlorhypoxanthin

20 g 2-Chlorhypoxanthin-Natrium-Hydrat werden in 400 ml Wasser suspendiert, mit verdünnter Salzsäure wird in der Reaktionslösung der pH-Wert auf 6,5 eingestellt und die Mischung auf ca. 60°C erwärmt. Nach Behandlung mit 2 g Entfärbungskohle und Filtration wird das 2-Chlorhypoxanthin, in gleicher Weise wie im Beispiel 2 beschrieben (bei pH 3,0 +/- 0,5 und Raumtemperatur) gefällt und isoliert. Man erhält 11,6 g (81 % d. Th.) der Titelverbindung in Form farbloser Kristalle. Das Produkt weist nach HPLC-Analyse eine Reinheit von über 99 % auf.

## Patentansprüche

1. Verfahren zur Herstellung und Reinigung von 2-Chlor-1,7-dihydropurin-6-on (2-Chlorhypoxanthin), dadurch gekennzeichnet, daß man 2-Thioxanthin in Salzsäure suspendiert und in die Suspension Chlor einleitet, die salzsaure Reaktionsmischung einengt, den Rückstand abkühlt, die Kristalle des entstandenen 2-Chlorhypoxanthin-Hydrochlorid-Monohydrates isoliert, anschließend in Wasser suspendiert, in der Suspension mit der Lösung eines Alkali- oder Erdalkalihydroxids einen pH-Wert im Bereich von pH 6 bis 7 einstellt, die Suspension auf eine Temperatur im Bereich von 50°C bis 70°C erwärmt, von festen Bestandteilen befreit und in der wäßrigen Lösung des Filtrates mit der Lösung einer Säure einen pH-Wert im Bereich von pH 2 bis pH 4 aufrechterhält, die dabei resultierende Suspension auf eine Temperatur im Bereich von -5°C bis +15°C abkühlt und die Kristalle des 2-Chlorhypoxanthins isoliert.

2. Verfahren nach Anspruch 1, dadurch gekennzeichnet, daß man 2-Thioxanthin in Salzsäure suspendiert und in die Suspension Chlor einleitet, die salzsaure Reaktionsmischung einengt, den Rückstand abkühlt, die Kristalle des entstandenen 2-Chlorhypoxanthin-Hydrochlorid-Monohydrates isoliert, anschließend in Wasser suspendiert, in der Suspension mit der Lösung eines Alkalihydroxids einen pH-Wert im Bereich von pH 6 bis pH 7 einstellt, die Suspension auf eine Temperatur im Bereich von 55°C bis 65°C erwärmt, anschließend filtriert und in der wäßrigen Lösung des Filtrates mit der wäßrigen Lösung einer Mineralsäure einen pH-Wert im Bereich von pH 2 bis pH 4 aufrechterhält, die dabei resultierende Suspension auf eine Temperatur im Bereich von -5 bis +15°C abkühlt und die Kristalle des 2-Chlorhypoxanthins isoliert.

3. Verfahren nach Anspruch 1, dadurch gekennzeichnet, daß man 2-Thioxanthin in Salzsäure suspendiert und in die Suspension Chlor einleitet, die salzsaure Reaktionsmischung einengt, den Rückstand abkühlt, die Kristalle des entstandenen 2-Chlorhypoxanthin-Hydrochlorid-Monohydrates isoliert, anschließend in Wasser suspendiert, in der Suspension mit Natronlauge einen pH-Wert von pH 6,5 einstellt, die Suspension auf eine Temperatur von 60°C erwärmt, anschließend filtriert - gegebenenfalls unter Zusatz eines Filterhilfsmittels - und mit Salzsäure in dem Filtrat mit verdünnter Salzsäure einen pH-Wert von pH 3 einstellt, die dabei resultierende Suspension auf eine Temperatur im Bereich von 5°C bis 10°C abkühlt und die Kristalle des 2-Chlorhypoxanthins isoliert.

4. Verfahren zur Reinigung von 2-Chlorhypoxanthin, dadurch gekennzeichnet, daß man das nach einem der Ansprüche 1 bis 3 hergestellte 2-Chlorhypoxanthin-Hydrochlorid-Monohydrat in Wasser suspendiert, mit einer anorganischen Base in der Suspension einen pH-Wert im Bereich von pH 9 bis pH 10 einstellt, anschließend die Reaktionsmischung auf eine Temperatur unterhalb der Raumtemperatur abkühlt, den dabei resultierenden Niederschlag isoliert und mit einem organischen Lösungsmittel wäscht, die Kristalle trocknet und anschließend in Wasser suspendiert, in der Suspension mit einer Säure einen pH-Wert im Bereich von pH 6 bis pH 7 einstellt, die Reaktionsmischung auf eine Temperatur in einem Intervall von 55°C bis 65°C erwärmt, die Lösung von festen Bestandteilen befreit und in der wäßrigen Lösung des Filtrates, mit einer Säure einen pH-Wert im Bereich von pH 2 bis pH 4 aufrechterhält, die resultierende Suspension auf eine Temperatur unterhalb Raumtemperatur abkühlt, den dabei resultierenden Niederschlag absaugt, mit einem organischen Lösungsmittel wäscht, und die Kristalle trocknet.

5. Verfahren zur Reinigung von 2-Chlorhypoxanthin nach Anspruch 4, dadurch gekennzeichnet, daß man das nach einem der Ansprüche 1 bis 3 hergestellte 2-Chlorhypoxanthin-Hydrochlorid-Monohydrat in Wasser suspendiert, mit einer Lösung eines Alkalihydroxids in der Suspension einen pH-Wert im Bereich von pH 9,5 bis pH 10 einstellt, anschließend die Reaktionsmischung auf eine Temperatur in einem Intervall von -2°C bis +10°C abkühlt, den dabei resultierenden Niederschlag isoliert und mit einem Alkohol wäscht, die Kristalle trocknet und anschließend in Wasser suspendiert, in der Suspension mit einer Mineralsäure einen pH-Wert im Bereich pH 6 bis pH 7 einstellt, die Reaktionsmischung auf eine Temperatur im Intervall von 55°C bis 65°C erwärmt, die Lösung von festen Bestandteilen befreit und in der wäßrigen Lösung des Filtrates mit der wäßrigen Lösung einer Mineralsäure einen Bereich von pH 2 bis pH 4 aufrechterhält, die resultierende Suspension auf eine Temperatur im Intervall von -2°C bis +15°C abkühlt, den dabei resultierenden Niederschlag absaugt, mit einem Alkohol wäscht und die Kristalle trocknet.

6. Verfahren zur Reinigung von 2-Chlorhypoxanthin in nach Anspruch 4, dadurch gekennzeichnet, daß man das nach einem der Ansprüche 1 bis 3 hergestellte 2-Chlorhypoxanthin-Hydrochlorid-Monohydrat in Wasser suspendiert mit Natronlauge in der Suspension einen pH-Wert von 9,7 einstellt, anschließend die Reaktionsmischung auf eine Temperatur im Bereich von 2°C bis 5°C abkühlt, den dabei resultierenden Niederschlag absaugt und mit Isopropanol wäscht, die Kristalle trocknet und anschließend in Wasser suspendiert, in der Suspension mit wäßriger Salzsäure einen pH-Wert von 6,5 einstellt, die Reaktionsmischung auf eine Temperatur von 60°C erwärmt, die Lösung - gegebenenfalls nach Behandlung mit Aktivkohle - filtriert und in der wäßrigen Lösung des Filtrates einen pH-Wert von pH 3 aufrechterhält, die resultierende Suspension auf eine Temperatur im Intervall von 5°C bis 10°C abkühlt, den dabei resultierenden Niederschlag absaugt mit Isopropanol wäscht und die Kristalle trocknet.

## Claims

1. Process for the preparation and purification of 2-chloro-1,7-dihydropurin-6-one (2-chlorohypoxanthine), characterised in that 2-thioxanthine is suspended in hydrochloric acid and chlorine is introduced into the suspension, the hydrochloric acid reaction mixture is concentrated by evaporation, the residue is cooled, the crystals of the resulting 2-chlorohypoxanthine-hydrochloride-monohydrate are isolated, then suspended in water, the pH of the suspension is adjusted to a pH in the range from 6 to 7 with a solution of an alkali metal hydroxide or alkaline earth metal hydroxide, the suspension is heated to a temperature in the range from 50°C to 70°C, solid constituents are eliminated and a pH in the range from 2 to 4 is maintained in the aqueous solution of the filtrate by means of a solution of an acid, the resulting suspension is cooled to a temperature in the range from -5°C to +15°C and the crystals of the 2-chlorohypoxanthine are isolated.

2. Process according to claim 1, characterised in that 2-thioxanthine is suspended in hydrochloric acid and chlorine is introduced into the suspension, the hydrochloric acid reaction mixture is concentrated by evaporation, the residue is cooled, the crystals of the resulting 2-chlorohypoxanthine-hydrochloride-monohydrate are isolated, then suspended in water, the pH of the suspension is adjusted to a pH in the range from 6 to 7 using a solution of an alkali metal hydroxide, the suspension is heated to a temperature in the range from 55°C to 65°C, then filtered, and a pH in the range from 2 to 4 is maintained in the aqueous solution of the filtrate using an aqueous solution of a mineral acid, the resulting suspension is cooled to a temperature in the range from -5 to +15°C and the crystals of the 2-chlorohypoxanthine are isolated.

3. Process according to claim 1, characterised in that 2-thioxanthine is suspended in hydrochloric acid and chlorine is introduced into the suspension, the hydrochloric acid reaction mixture is concentrated by evaporation, the residue is cooled, the crystals of the resulting 2-chlorohypoxanthine-hydrochloride-monohydrate are isolated, then suspended in water, the pH of the suspension is adjusted to 6.5 using sodium hydroxide solution, the suspension is heated to a temperature of 60°C, then filtered - optionally with the addition of a filter aid - and the pH of the filtrate is adjusted to 3 with hydrochloric acid and dilute hydrochloric acid, the resulting suspension is cooled to a temperature in the range from 5°C to 10°C and the crystals of the 2-chlorohypoxanthine are isolated.

4. Process for the purification of 2-chlorohypoxanthine, characterised in that 2-chlorohypoxanthine-hydrochloride-monohydrate prepared according to one of claims 1 to 3 is suspended in water, the pH of the suspension is adjsted to a pH in the range from 9 to 10 using an inorganic base, then the reaction mixture is cooled to a temperature below ambient temperature, the resulting precipitate is isolated and washed with an organic solvent, the crystals are dried and then suspended in water, the pH of the suspension is adjusted to a pH in the range from 6 to 7 using an acid, the reaction mixture is heated to a temperature in the range from 55°C to 65°C, any solid constituents are removed from the solution and a pH in the range from 2 to 4 is maintained in the aqueous solution of the filtrate using an acid, the resulting suspension is cooled to a temperature below ambient temperature, the resulting precipitate is suction filtered, washed with an organic solvent, and the crystals are dried.

5. Process for the purification of 2-chlorohypoxanthine according to claim 4, characterised in that the 2-chlorohypoxanthine-hydrochloride-monohydrate prepared according to one of claims 1 to 3 is suspended in water, the pH of the suspension is adjusted to a pH in the range from 9.5 to 10 by means of a solution of alkali metal hydroxide, then the reaction mixture is cooled to a temperature in the range from -2°C to +10°C, the resulting precipitate is isolated and washed with an alcohol, the crystals are dried and then suspended in water, the pH of the suspension is adjusted to a pH in the range from 6 to 7 using a mineral acid, the reaction mixture is heated to a temperature in the range from 55°C to 65°C, any solid constituents are removed from the solution and a pH in the range from 2 to 4 is maintained in the aqueous solution of the filtrate using an aqueous solution of a mineral acid, the resulting suspension is cooled to a temperature in the range from -2°C to +15°C, the resulting precipitate is suction filtered, washed with an alcohol and the crystals are dried.

6. Process for the purification of 2-chlorohypoxanthine according to claim 4, characterised in that the 2-chlorohypoxanthine-hydrochloride-monohydrate prepared according to one of claims 1 to 3 is suspended in water, the pH of the suspension is adjusted to 9.7 using sodium hydroxide solution, then the reaction mixture is cooled to a temperature in the range from 2°C to 5°C, the resulting precipitate is suction filtered and washed with isopropanol, the crystals are dried and then suspended in water, the pH of the suspension is adjusted to 6.5 using aqueous hydrochloric acid, the reaction mixture is heated to a temperature of 60°C, the solution is filtered, optionally after treatment with activated charcoal, and a pH of 3 is maintained in the aqueous solution of the filtrate, the resulting suspension is cooled to a temperature in the range from 5°C to 10°C, the resulting precipitate is suction filtered and washed with isopropanol and the crystals are dried.

## Revendications

1. Procédé de préparation et de purification de la 2-chloro-1,7-dihydropurin-6-one (2-chlorohypoxanthine), caractérisé en ce que l'on met en suspension la 2-thioxanthine dans l'acide chlorhydrique et on introduit du chlore dans la suspension, on concentre le mélange réactionnel chlorhydrique, on refroidit le résidu, on isole les cristaux du monohydrate de chlorhydrate de 2-chlorohypoxanthine formé, puis on les met en suspension dans l'eau, on établit dans la suspension un pH situé dans le domaine de pH 6 à 7 avec la solution d'un hydroxyde alcalin ou alcalino-terreux, on chauffe la suspension à une température située dans le domaine de 50°C à 70°C, on la débarrasse des constituants solides et on maintient dans la solution aqueuse du filtrat un pH situé dans le domaine de pH 2 à pH 4 avec la solution d'un acide, on refroidit la suspension résultante à une température située dans le domaine de -5°C à +15°C et on isole les cristaux de 2-chlorohypoxanthine.

2. Procédé selon la revendication 1, caractérisé en ce que l'on met en suspension la 2-thioxanthine dans l'acide chlorhydrique et on introduit du chlore dans la suspension, on concentre le mélange réactionnel chlorhydrique, on refroidit le résidu, on isole les cristaux de monohydrate de chlorhydrate de 2-chlorohypoxanthine formé, puis on les met en suspension dans l'eau, on établit dans la suspension un pH situé dans le domaine de pH 6 à pH 7 avec la solution d'un hydroxyde alcalin, on chauffe la suspension à une température située dans le domaine de 55°C à 65°C, puis on filtre et on maintient dans la solution aqueuse du filtrat un pH situé dans le domaine de pH 2 à pH 4 avec la solution aqueuse d'un acide minéral, on refroidit la suspension résultante à une température située dans le domaine de -5 à +15°C et on isole les cristaux de 2-chloro-hypoxanthine.

3. Procédé selon la revendication 1, caractérisé en ce que l'on met en suspension la 2-thioxanthine dans l'acide chlorhydrique et on introduit du chlore dans la suspension, on concentre le mélange réactionnel chlorhydrique, on refroidit le résidu, on isole les cristaux du monohydrate de chlorhydrate de 2-chlorohypoxanthine formé, puis on les met en suspension dans l'eau, on établit dans la suspension un pH de 6,5 de la lessive de soude, on chauffe la suspension à une température de 60°C, puis on filtre, en ajoutant éventuellement un adjuvant de filtration, et on maintient dans le filtrat un pH de 3 avec de l'acide chlorhydrique dilué, on refroidit la suspension résultante à une température située dans le domaine de 5°C à 10°C et on isole les cristaux de 2-chlorohypoxanthine.

4. Procédé de purification de la 2-chlorohypoxanthine, caractérisé en ce que l'on met en suspension dans l'eau le monohydrate de chlorhydrate de 2-chlorohypoxanthine préparé selon l'une des revendications 1 à 3, on établit dans la suspension un pH situé dans le domaine de pH 9 à pH 10 avec une base inorganique, puis on refroidit le mélange réactionnel à une température inférieure à la température ambiante, on isole le précipité résultant et on le lave avec un solvant organique, on sèche les cristaux puis on les met en suspension dans l'eau, on établit dans la suspension un pH situé dans le domaine de pH 6 à pH 7 avec un acide, on chauffe le mélange réactionnel à une température située dans un intervalle de 55°C à 65°C, on débarrasse la solution des constituants solides et on maintient dans la solution aqueuse du filtrat un pH situé dans le domaine de pH 2 à pH 4 avec un acide, on refroidit la suspension résultante à une température inférieure à la température ambiante, on essore le précipité résultant, on le lave avec un solvant organique et on sèche les cristaux.

5. Procédé de purification de la 2-chlorohypoxanthine selon la revendication 4, caractérisé en ce que l'on met en suspension dans l'eau le monohydrate de chlorhydrate de 2-chlorohypoxanthine préparé selon l'une des revendications 1 à 3, on établit dans la suspension un pH situé dans le domaine de pH 9,5 à pH 10 avec une solution d'un hydroxyde alcalin, puis on refroidit le mélange réactionnel à une température située dans un intervalle de -2°C à +10°C, on isole le précipité résultant et on le lave avec un alcool, on sèche les cristaux puis on les met en suspension dans l'eau, on établit dans la suspension un pH situé dans le domaine de pH 6 à pH 7 avec un acide minéral, on chauffe le mélange réactionnel à une température située dans un intervalle de 55°C à 65°C, on débarrasse la solution des constituants solides et on maintient dans la solution aqueuse du filtrat un pH situé dans le domaine de pH 2 à pH 4 avec la solution aqueuse d'un acide minéral, on refroidit la suspension résultante à une température située dans l'intervalle de -2°C à +15°C, on essore le précipité résultant, on le lave avec un alcool et on sèche les cristaux.

6. Procédé de purification de la 2-chlorohypoxanthine selon la revendication 4, caractérisé en ce que l'on met en suspension dans l'eau le monohydrate de chlorhydrate de 2-chlorohypoxanthine préparé selon l'une des revendications 1 à 3, on établit dans la suspension un pH de 9,7 avec de la lessive de soude, puis on refroidit le mélange réactionnel à une température située dans le domaine de 2°C à 5°C, on essore le précipité résultant et on le lave avec de l'isopropanol, on sèche les cristaux puis on les met en suspension dans l'eau, on établit dans la suspension un pH de 6,5 avec de l'acide chlorhydrique aqueux, on chauffe le mélange réactionnel à une température de 60°C, on filtre la solution, éventuellement après traitement avec du charbon actif, et on maintient dans la solution aqueuse du filtrat un pH de 3, on refroidit la suspension résultante à une température située dans l'intervalle de 5°C à 10°C, on essore le précipité résultant, on le lave avec de l'isopropanol et on sèche les cristaux.
